# EUROPEAN PATENT APPLICATION

(11) **EP 0 866 125 A1**
(43) Date of publication of application: **23.09.1998**
(21) Application number: 96925064.6
(22) Date of filing: 24.07.1996
(51) Int. Cl.: C12N 15/12, C07K 14/51, C12P 21/02, C12N 5/10, A61K 38/17

(54) **NOVEL PROTEIN HMW HUMAN MP52**

(30) Priority: 24.07.1995 JP 218022/95
(71) Applicant: Hoechst Marion Roussel, Ltd., Tokyo 107-8465 (JP)
(72) Inventor: KIMURA, Michio, Nippon Hoechst Marion Roussel Ltd., Kawagoe-shi, Saitama 350-11 (JP); MATSUMOTO Tomoaki, Nippon Hoechst Marion Rssl Ltd., Kawagoe-shi, Saitama 350-11 (JP); TAKAHASHI Mikiko, Nippon Hoechst Marion Rssl Ltd., Kawagoe-shi, Saitama 350-11 (JP); KAWAI, Shinji, Nippon Hoechst Marion Roussel Ltd., Kawagoe-shi, Saitama 350-11 (JP); FUJINO, Yukio, Nippon Hoechst Marion Roussel Ltd., Kawagoe-shi, Saitama 350-11 (JP)
(74) Representative: Vieillefosse, Jean-Claude
(86) International application number: JP9602065
(87) International publication number: WO9704095

(57) **Abstract**

The present invention provides proteins named HMW human MP52s having some amino acid sequence of SEQ ID NO.:1 which are produced in CHO cells. The present invention also provides a process for the production of HMW human MP52s and a pharmaceutical composition containing as an active ingredient HMW human MP52s.

HMW human MP52s can be used for treating or preventing bone diseases, *etc*. due to their property of promoting bone morphogenesis.

## Description

### FIELD OF THE INVENTION

This invention relates to new HMW human MP52s and a medical composition *inter alia* for promoting cartilage and bone morphogenesis comprising HMW human MP52s. In particular, the medical composition is useful for treating bone diseases caused by abnormal bone metabolism such as osteoporosis, for treating bone fracture and for the purpose of orthopedic reconstruction, bone transplantation, cosmetic surgery and dental therapeutics. Further, it is useful for treating cartilage disorders.

### DESCRIPTION OF THE PRIOR ART

Pharmaceutical compositions including vitamin D₃, calcitonin, estrogen and bisphosphonate derivatives have been used in clinical practice for treating bone diseases. Their therapeutic results, however, are not entirely satisfactory, and a better pharmaceutical composition is highly desired.

The TGF-β superfamily of growth factors such as BMP, TGF, and inhibin related proteins have been reported to be useful for wound healing and tissue repair. The bone morphogenetic activity of some of those proteins has been also known. PCT patent application WO 93/16099 and WO 95/04819 disclosed DNA sequences encoding human TGF-β-like proteins, and as a preferred protein human MP52.

E. E. Storm et al. reported in Nature, 1994, vol. 368, p.639-642 that three mouse growth/differentiation factors, i.e. GDF5, GDF6 and GDF7 were identified as new members of TGF-β superfamily and that the mutations in GDF5 gene caused mice brachypodism. Mouse GDF5 has the same amino acid sequence of the predicted mature form as that of human MP52 except one amino acid. However, there is no suggestion in this publication to use those proteins for the treatment of bone diseases.

### DETAILED DESCRIPTION OF THE INVENTION

It was therefore the object of the present invention to provide a further growth factor that is useful as an agent for the stimulation of bone or cartilage formation.

Mature MP52 is considered as a protein having 120 amino acids. Its amino acid sequence is from 355 to 474 of SEQ ID NO.:1 of the Sequence Listing (WO 95/04819). In fact, it has surprisingly been found by the inventors that there are cell lines which, when expressing a suitable DNA sequence, form proteins having various length of amino acid sequence, namely mature MP52 and HMW (high molecular weight) human MP52s. For the first time the invention succeeded in proving HMW human MP52s which have bone morphogenetic activity and are useful for preventing and/or treating bone diseases.

The present invention relates to HMW human MP52s which fall under any one of the following definitions:
(1) a dimeric protein comprising peptides having amino acid sequence from 1 to 474 of SEQ ID NO.:1.
(2) a dimeric protein comprising peptides having amino acid sequence from 121 to 474 of SEQ ID NO.:1.
(3) a dimeric protein comprising peptides having amino acid sequence from 122 to 474 of SEQ ID NO.:1.
(4) a dimeric protein comprising a peptide having amino acid sequence from 121 to 474 of SEQ ID NO.:1 and a peptide having amino acid sequence from 122 to 474 of SEQ ID NO.:1.
(5) a dimeric protein comprising a peptide having amino acid sequence from 1 to 474 of SEQ ID NO.:1 and a peptide having amino acid sequence from 121 and/or 122 to 474 of SEQ ID NO.:1.
(6) a dimeric protein comprising a peptide having amino acid sequence from 1 to 474 of SEQ ID NO.:1 and a peptide having amino acid sequence from 355 to 474 of SEQ ID NO.:1.
(7) a dimeric protein comprising a peptide having amino acid sequence from 121 and/or 122 to 474 of SEQ ID NO.:1 and a peptide having amino acid sequence from 355 to 474 of SEQ ID NO.:1.

The proteins usually exist as dimers through -S-S- bonds formed by cysteins. The peptide in this specification means a monomer.

HMW human MP52s induce formation of cartilage from undifferentiated mesenchymal cells and stimulates the differentiation and maturation of osteoblasts. Therefore, HMW human MP52s are effective for preventing and/or treating bone diseases caused by abnormal bone metabolism such as osteoporosis. They also accelerates the healing process of bone fractures. Moreover, they are useful for orthopedic reconstruction, bone transplantations and dental therapeutics because of its bone morphogenetic activity. Furthermore, HMW human MP52s are effective for preventing and/or treating cartilage disorders caused by abnormal cartilage metabolism.

A further object of the present invention is to provide a process for the production of HMW human MP52s, wherein DNA sequence coding for HMW human MP52s as shown in SEQ ID NO.:1 is introduced into a suitable host cell and cultivating it under conditions favoring expression of the DNA sequence and protein formation, followed by isolation of said protein from other proteins recovered from said host cell.

Within the framework of the present invention the DNA sequence depicted in SEQ ID NO.:1 may be used for producing HMW human MP52s, however, also shorter portions thereof, provided they still encode HMW human MP52s and expression of the DNA sequence in a suitable vector/host cell system is possible. Suitable expression systems are known to a person skilled in the art and it can easily be determined by routine experimentation what the minimum requirements for the length of the DNA sequence of SEQ ID NO.:1. are.

Subsequent to protein formation the proteins are recovered from the host cell by methods known per se and finally HMW human MP52s are isolated therefrom. In particular the isolation of HMW human MP52s can be carried out using very precisely differentiating separation methods which are known to a person skilled in the art. For example, reverse phase HPLC might be considered to this end.

A further object of the present invention is to provide a pharmaceutical composition containing HMW human MP52s. Optionally, this composition may also include usual carrier substances, auxiliary substances, diluents and/or fillers. The pharmaceutical composition according to the invention is useful for promoting bone morphogenation, treatment or prevention of damage to bone, cartilage, connective tissues, skin, mucous membranes, epithelium or teeth, for application in dental implants and for application in wound-healing and tissue regeneration processes.

It is possible to administer one of HMW human MP52s separately or in a form of a mixture of HMW human MP52s.

For the treatment of bone diseases caused by abnormal bone metabolism, HMW human MP52s are administered in systemic by injection such as intravenous injection, intramusclar injection and intraperitoneal injection, oral administration, parenteral administration such as suppository, and any other conventional methods.

For the treatment of bone fracture, they are administered in systemic and locally by injection, oral and parenteral administration. Also matrixes containing HMW human MP52s are preferably implanted in the area close to the fractured bone. Suitable matrixes are natural polymers such as collagen and fibrin glue, and artificial polymers degradable in living body such as poly lactated glycolic acid.

In case of orthopedic reconstruction, cosmetic surgery, bone transplantation and dental implantation, HMW human MP52s for example can be coated on the surface of bone and tooth to be implanted by means of collagen paste, fibrin glue and other adhering materials. It can also be applied to the tissue, the bone or alveolar bone around which the bone and tooth is transplanted. In case of bone transplantation it can be used for both natural and artificial bone. As the material of artificial bone and tooth, conventional materials such as metals, ceramics, glass and other natural or artificial inorganic substances are used. Hydroxyapatite is a preferable artificial substance. Artificial bone can be constructed by dense material in the inner part and porous material in the other part. For example, dense steel covered porous steel can be cited. Porous hydroxyapatite is one of the materials to produce artificial bone. When such porous material is used, HMW human MP52s can be penetrated into it. The surface of artificial bone can be roughened to keep HMW human MP52s on the surface.

It is also beneficial to apply HMW human MP52s to the part from which cancerous bone tissue is removed in order to accelerate the reconstruction of bone.

The dose of HMW human MP52s to be administered is decided depending upon the purpose and the method of application. In general, when it is administered in systemic, the dose is from 1 µg to 100 µg/kg. When it is used implantation, the preferred dose is from 30 µg to 30 mg per site.

These purified HMW human MP52s can be formulated in any conventional forms such as injection liquid, pills, capsules and suppository. For local administration HMW human MP52s can be included in a matrix such as collagen, fibrin glue and poly lactated glycolic acid. For the use of implantation and transplantation it is applied to the surface or in the porous part of bone and tooth.

### BRIEF DESCRIPTION OF DRAWINGS

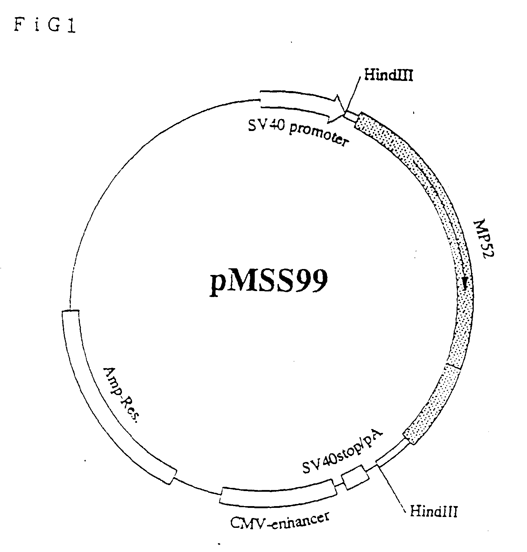

Fig. 1 shows a plasmid map of a HMW human MP52s expression vector pMSS99 (5.0 kb). The HMW human MP52s DNA sequence in pMSS99 is the nucleotides from the 576th to the 2279th shown in the SEQ ID NO:1 of the Sequence Listing.

### DESCEIPTION OF THE PREFERRED EMBODIMENTS

This invention is illustrated by the examples.

### Example 1

### Production of HMW human MP52s

### (1) Construction of expression vector for HMW human MP52s

The pSK52s vector containing human MP52 gene, which was supplied by Dr. Hötten of Biopharm GmbH, was digested with *Hin*d III and DNA fragment containing human MP52 gene was isolated by the extraction from 0.8 % low melting agarose gels and ligated into the *Hin*d III site of pAB stop vector, which is supplied by Dr. Gerd Zettlmeissl of Behringwerke AG. The structure of the HMW human MP52s expression vector, pMSS99 (5.0 kb) as shown in Fig. 1, was confirmed by the DNA sequencing and the restriction enzyme mapping. The HMW human MP52s DNA sequence in pMSS99 was the nucleotides from the 576th to the 2279th shown in the SEQ ID NO:1 of the Sequence Listing.

### (2) Establishment of CHO clones producing HMW human MP52s

CHO-DUKX-B11 cells, mutants of CHO cells, which were provided by Dr. Zettlmeissl of Behringwerke AG, were co-transfected with pMSS99 and pSVOAdhfr which was also provided by Dr. Zettlmeissl, by calcium phosphate-mediated DNA transfer method. Then high producer clones of HMW human MP52s were established by gene amplification protocol using methotrexate (MTX).

Ten µg of pMSS99 and 2 µg of pSV0Adhfr were dissolved in 1 ml of 25 mM HEPES-140 mM NaCl-0.75 mM Na₂HPO₄ (pH 7.05), then mixed with 50 µl of 2.5 M CaCl₂. The resultant precipitates were overlaid to CHO-DUKX-B11 cells in a 10 cm dish and stood at room temperature for 30 min. Then 8 ml of MEM ALPHA with ribo- and deoxyribo-nucleosides (MEMα⁺) containing 10% fetal bovine serum (FBS) was added to the cell layer to incubate in a CO₂ incubator for 4-6 h. After the treatment with 10% glycerol in MEMα⁺ containing 10% FBS at room temperature for 3 min, the cells were cultured in MEMα⁺ containing 10% FBS for 2 days. Then the cells were replaced in MEM ALPHA without ribo- and deoxyribo-nucleosides (MEMα⁻) containing 10% dialyzed FBS to select the transformants. The transformant clones were isolated and assayed for the expression of HMW human MP52s by Western blot analysis as described in the next session.

The HMW human MP52s producing clones were further selected stepwisely in increasing concentrations of methotrexate (MTX) to amplify the MP52 gene in accordance with the pSVOAdhfr gene. Several clones were obtained to produce 1-3 µg of HMW human MP52s/10⁶ cells/24 h at 400 nM MTX.

### (3) Detection of HMW human MP52s in the culture supernatants

Clones were examined for the expression of HMW human MP52s by Western blot analysis as follows: the culture supernatants (1-15 µl) were applied on SDS-PAGE (15-25% polyacrylamide gradient gel, Daiichi Pure Chemicals) under reducing condition, then the proteins were transferred to a PVDF membrane (Clear Blot Membrane-P, ATTO). The membrane was blocked with Block Ace (Dai-Nihon Seiyaku) for 1 h, rinsed with Tris-buffered saline (TBS), then treated with 10 µg/ml of chicken antibodies to HMW human MP52s in 10-times diluted Block Ace overnight. After washing the membrane with 0.1% Tween 20 in TBS (TTBS), the membrane was treated with rabbit anti-chicken IgG-ALP conjugate (Sigma A 9171) in 10-times diluted Block Ace for 1 h. The membrane was washed with TTBS. then, reacted with Alkaline phosphatase Conjugate Substrate Kit (BIO-RAD) to visualize the bands corresponding to MP52.

### (4) Cell culture of the HMW human MP52s-producing CHO cell line

The CHO cell line with the highest productivity of HMW human MP52s, MC-2 (Deposit No. FERM BP-5142), was grown with roller bottles containing MEMα- supplemented with 10% FBS, 400 nM MTX, 100 U/ml Penicillin, 100 µg/ml Streptomycin. After the MC-2 cells were up to confluency, they were washed with serum-free MEMα- and then cultured in serum-free DME/F12 supplemented with 10 mM HEPES (pH 7.3), 10 KIU Aprotinin, 1 mM sodium butyrate, 6 µg/ml sodium selenate, 5 µg/ml transferrin, 18 µg/ml ethanol amine, 9 µg/ml insulin, 100 U/ml Penicillin, 100 µg/ml Streptomycin. The conditioned medium was collected every day for a week.

### (5) Purification of HMW human MP52s

The CHO culture supernatant and 0.1 vol. of 0.2 M sodium phosphate buffer, pH 6.0, were mixed and applied to POROS HS column (10 ml, PerSeptive Biosystems) previously equilibrated with 50 mM NaCl, 20 mM sodium phosphate buffer, pH 6.0. The proteins were eluted by linear gradient of NaCl from 0.05 to 2 M and collected with 20 of 10 ml fractions. The eluted MP52s were observed as three types of monomers and their apparent molecular weights were determined about 52, 40 and 14 kD by SDS-PAGE analysis under reduced condition. These monomers form three types of homodimers (104 kD, 80 kD and 28 kD) and three types of heterodimers (92 kD: 40 kD-52 kD, 66 kD: 14 kD-52 kD, and 54 kD: 14 kD-40 kD) and all those dimers were named HMW (high molecular weight) human MP52s except 28 kD homodimer which seemed to be known as a mature homodimer of human MP52 (WO 95/04819). Therefore, 104 kD homodimer and 80 kD homodimer were isolated from the above fractions to examine the N-terminal amino acid sequences and the biological activities.

The fractions from 5th to 9th were pooled and concentrated about 10 fold. The concentrate was loaded to Superdex 200 pg (1.6 I.D. x 60 cm. Pharmacia) previously equilibrated with 20 mM sodium phosphate buffer, pH 7.1, containing 1 M NaCl. The elution was performed at the flow rate of 0.5 ml/min. The fractions containing the 104 kD homodimer and the fractions containing the 80 kD homodimer were pooled separately. Each was applied to reverse phase HPLC column (RESOURCE RPC, 3 ml, Pharmacia) and these were eluted at 35-40 % acetonitrile. The concentrations of the isolated HMW human MP52s were determined by densitometory of the bands of proteins on SDS-PAGE gel.

The N-terminal amino acid sequence analysis was performed using a pulse liquid gas phase sequencer (Applied Biosystems model 476) for 80 kD homodimer and 104 kD homodimer, respectively. The results are shown in table 1.

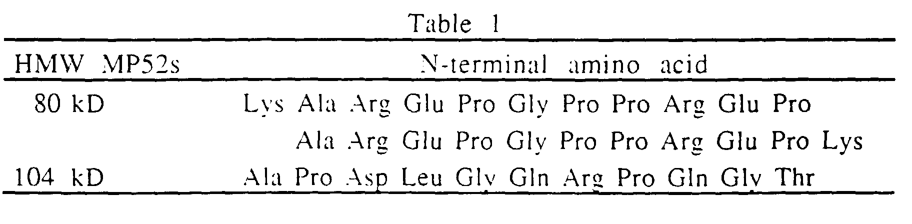

The amino acid sequences 80 kD were from Lys 121 or Ala 122 to Arg 474 and that of 104 kD was from Ala 1 to Arg 474 in SEQ ID NO.:1 of the Sequence Listing. It was newly found that the CHO cells produced 3 types of homodimers, 104 kD, 80 kD and 28 kD, and 3 types of heterodimers, namely dimers of 92 kD, 66 kD and 54 kD.

### Example 2

### Biological activity

Osteoprogenitor-like ROB-C26 cells (Calcif. Tissue Int. vol. 49, pp. 221-225, 1991) cloned from newborn rat calvarial cells were plated onto 48-well multi-well plates (Coaster) at a density of 1.5 x 10⁴ cells/well and pre-incubated for 3 days in MEMα⁻ containing 10% FBS. After the removal of culture medium, fresh MEMα⁻containing 10% FBS and serially diluted 80 kD or 104 kD HMW human MP52s in 10 mM HCl was added to the cultures and incubated for 6 days with changing the medium and the additives on day 3. Cell layers were washed with phosphate-buffered saline, and extracted with 0.2% Nonidet containing 1 mM MgCl₂. Alkaline phosphatase (ALP) activities were determined according to the procedure of Takuwa et al. (Am. J. Physiol. vol. 257, p. E797-E803, 1989). As shown in Table 2, the treatment of ROB-C26 cells with either 80 kD or 104 kD HMW human MP52s increased total ALP activities per well concentration-dependently.

**Table 2**

| Influence of 80 kD and 104 kD HMW human MP52s on the ALP activity of the ROB-C26 cell line. | | |
|---|---|---|
| Compound | Concentration (ng/ml) | ALP activity (nmol/min/well) |
| Vehicle (10 mM HCl)-treated control | - | 5.47 ± 0.81 |
| 80 kD HMW human MP52 | 8.6 | 5.42 ± 1.09 |
| | 29 | 7.00 ± 0.89 |
| | 86 | 14.30 ± 0.24* |
| | 290 | 16.28 ± 0.19* |
| | 860 | 18.41 ± 1.95* |
| 104 kD HMW human MP52 | 11 | 6.51 ± 0.90 |
| | 38 | 7.54 ± 0.29 |
| | 110 | 8.32 ± 0.12* |
| | 380 | 12.07 ± 0.53* |
| | 1100 | 16.98 ± 0.47* |
| Values represent means ± S.D. of 3 or 4 cultures. | | |

| | | |
|---|---|---|
| *p<0.01 compared to the vehicle-treated control (Dunnett's test). | | |

"Sequence Listing"

SEQ ID NO:1
SEQUENCE TYPE:nucleotide with corresponding protein
SEQUENCE LENGTH:2703

STRANDEDNESS:double
TOPOLOGY:linear
MOLECULE TYPE:cDNA to mRNA

ORIGINAL SOURCE
ORGANISM:homo sapiens

FEATURES:
from 640 to 720 bp signal peptide
from 1783 to 2142 bp mature peptide

PROPERTIES:human embryo
SEQUENCE DESCRIPTION:SEQ ID NO:1:

CCATGGCCTC GAAAGGGCAG CGGTGATTTT TTTCACATAA ATATATCGCA CTTAAATGAG 60

TTTAGACAGC ATGACATCAG AGAGTAATTA AATTGGTTTG GGTTGGAATT CCGTTTCCAA 120

TTCCTGAGTT CAGGTTTGTA AAAGATTTTT CTGAGCACCT GCAGGCCTGT GAGTGTGTGT 180

GTGTGTGTGT GTGTGTGTGT GTGTGTGTGA AGTATTTTCA CTGGAAAGGA TTCAAAACTA 240

GGGGGAAAAA AAAACTGGAG CACACAGGCA GCATTACGCC ATTCTTCCTT CTTGGAAAAA 300

TCCCTCAGCC TTATACAAGC CTCCTTCAAG CCCTCAGTCA GTTGTGCAGG AGAAAGGGGG 360

CGGTTGGCTT TCTCCTTTCA AGAACGAGTT ATTTTCAGCT GCTGACTGGA GACGGTGCAC 420

GTCTGGATAC GAGAGCATTT CCACTATGGG ACTGGATACA AACACACACC CGGCAGACTT 480

CAAGAGTCTC AGACTGAGGA GAAAGCCTTT CCTTCTGCTG CTACTGCTGC TGCCGCTGCT 540

TTTGAAAGTC CACTCCTTTC ATGGTTTTTC CTGCCAAACC AGAGGCACCT TTGCTGCTGC 600

CGCTGTTCTC TTTGGTGTCA TTCAGCGGCT GGCCAGAGG ATG AGA CTC CCC AAA 654

Met Arg Leu Pro Lys

-25

CTC CTC ACT TTC TTG CTT TGG TAC CTG GCT TGG CTG GAC CTG GAA TTC 702

Leu Leu Thr Phe Leu Leu Trp Tyr Leu Ala Trp Leu Asp Leu Glu Phe

-20 -15 -10

ATC TGC ACT GTG TTG GGT GCC CCT GAC TTG GGC CAG AGA CCC CAG GGG 750

Ile Cys Thr Val Leu Gly Ala Pro Asp Leu Gly Gln Arg Pro Gln Gly

-5 1 5 10

ACC AGG CCA GGA TTG GCC AAA GCA GAG GCC AAG GAG AGG CCC CCC CTG 798

Thr Arg Pro Gly Leu Ala Lys Ala Glu Ala Lys Glu Arg Pro Pro Leu

15 20 25

GCC CGG AAC GTC TTC AGG CCA GGG GGT CAC AGC TAT GGT GGG GGG GCC 846

Ala Arg Asn Val Phe Arg Pro Gly Gly His Ser Tyr Gly Gly Gly Ala
30 35 40
ACC AAT GCC AAT GCC AGG GCA AAG GGA GGC ACC GGG CAG ACA GGA GGC 894
Thr Asn Ala Asn Ala Arg Ala Lys Gly Gly Thr Gly Gln Thr Gly Gly
45 50 55
CTG ACA CAG CCC AAG AAG GAT GAA CCC AAA AAG CTG CCC CCC AGA CCG 942
Leu Thr Gln Pro Lys Lys Asp Glu Pro Lys Lys Leu Pro Pro Arg Pro
60 65 70
GGC GGC CCT GAA CCC AAG CCA GGA CAC CCT CCC CAA ACA AGG CAG GCT 990
Gly Gly Pro Glu Pro Lys Pro Gly His Pro Pro Gln Thr Arg Gln Ala
75 80 85 90
ACA GCC CGG ACT GTG ACC CCA AAA GGA CAG CTT CCC GGA GGC AAG GCA 1038
Thr Ala Arg Thr Val Thr Pro Lys Gly Gln Leu Pro Gly Gly Lys Ala
95 100 105
CCC CCA AAA GCA GGA TCT GTC CCC AGC TCC TTC CTG CTG AAG AAG GCC 1086
Pro Pro Lys Ala Gly Ser Val Pro Ser Ser Phe Leu Leu Lys Lys Ala
110 115 120
AGG GAG CCC GGG CCC CCA CGA GAG CCC AAG GAG CCG TTT CGC CCA CCC 1134
Arg Glu Pro Gly Pro Pro Arg Glu Pro Lys Glu Pro Phe Arg Pro Pro
125 130 135
CCC ATC ACA CCC CAC GAG TAC ATG CTC TCG CTG TAC AGG ACG CTG TCC 1182
Pro Ile Thr Pro His Glu Tyr Met Leu Ser Leu Tyr Arg Thr Leu Ser
140 145 150
GAT GCT GAC AGA AAG GGA GGC AAC AGC AGC GTG AAG TTG GAG GCT GGC 1230
Asp Ala Asp Arg Lys Gly Gly Asn Ser Ser Val Lys Leu Glu Ala Gly
155 160 165 170
CTG GCC AAC ACC ATC ACC AGC TTT ATT GAC AAA GGG CAA GAT GAC CGA 1278
Leu Ala Asn Thr Ile Thr Ser Phe Ile Asp Lys Gly Gln Asp Asp Arg
175 180 185
GGT CCC GTG GTC AGG AAG CAG AGG TAC GTG TTT GAC ATT AGT GCC CTG 1326
Gly Pro Val Val Arg Lys Gln Arg Tyr Val Phe Asp Ile Ser Ala Leu
190 195 200

GAG AAG GAT GGG CTG CTG GGG GCC GAG CTG CGG ATC TTG CGG AAG AAG 1374
Glu Lys Asp Gly Leu Leu Gly Ala Glu Leu Arg Ile Leu Arg Lys Lys
205 210 215
CCC TCG GAC ACG GCC AAG CCA GCG GCC CCC GGA GGC GGG CGG GCT GCC 1422
Pro Ser Asp Thr Ala Lys Pro Ala Ala Pro Gly Gly Gly Arg Ala Ala
220 225 230
CAG CTG AAG CTG TCC AGC TGC CCC AGC GGC CGG CAG CCG GCC TCC TTG 1470
Gln Leu Lys Leu Ser Ser Cys Pro Ser Gly Arg Gln Pro Ala Ser Leu
235 240 245 250
CTG GAT GTG CGC TCC GTG CCA GGC CTG GAC GGA TCT GGC TGG GAG GTG 1518
Leu Asp Val Arg Ser Val Pro Gly Leu Asp Gly Ser Gly Trp Glu Val
255 260 265
TTC GAC ATC TGG AAG CTC TTC CGA AAC TTT AAG AAC TCG GCC CAG CTG 1566
Phe Asp Ile Trp Lys Leu Phe Arg Asn Phe Lys Asn Ser Ala Gln Leu
270 275 280
TGC CTG GAG CTG GAG GCC TGG GAA CGG GGC AGG GCC GTG GAC CTC CGT 1614
Cys Leu Glu Leu Glu Ala Trp Glu Arg Gly Arg Ala Val Asp Leu Arg
285 290 295
GGC CTG GGC TTC GAC CGC GCC GCC CGG CAG GTC CAC GAG AAG GCC CTG 1662
Gly Leu Gly Phe Asp Arg Ala Ala Arg Gln Val His Glu Lys Ala Leu
300 305 310
TTC CTG GTG TTT GGC CGC ACC AAG AAA CGG GAC CTG TTC TTT AAT GAG 1710
Phe Leu Val Phe Gly Arg Thr Lys Lys Arg Asp Leu Phe Phe Asn Glu
315 320 325 330
ATT AAG GCC CGC TCT GGC CAG GAC GAT AAG ACC GTG TAT GAG TAC CTG 1758
Ile Lys Ala Arg Ser Gly Gln Asp Asp Lys Thr Val Tyr Glu Tyr Leu
335 340 345
TTC AGC CAG CGG CGA AAA CGG CGG GCC CCA CTG GCC ACT CGC CAG GGC 1806
Phe Ser Gln Arg Arg Lys Arg Arg Ala Pro Leu Ala Thr Arg Gln Gly
350 355 360
AAG CGA CCC AGC AAG AAC CTT AAG GCT CGC TGC AGT CGG AAG GCA CTG 1854
Lys Arg Pro Ser Lys Asn Leu Lys Ala Arg Cys Ser Arg Lys Ala Leu
365 370 375

CAT GTC AAC TTC AAG GAC ATG GGC TGG GAC GAC TGG ATC ATC GCA CCC 1902
His Val Asn Phe Lys Asp Met Gly Trp Asp Asp Trp Ile Ile Ala Pro
380 385 390
CTT GAG TAC GAG GCT TTC CAC TGC GAG GGG CTG TGC GAG TTC CCA TTG 1950
Leu Glu Tyr Glu Ala Phe His Cys Glu Gly Leu Cys Glu Phe Pro Leu
395 400 405 410
CGC TCC CAC CTG GAG CCC ACG AAT CAT GCA GTC ATC CAG ACC CTG ATG 1998
Arg Ser His Leu Glu Pro Thr Asn His Ala Val Ile Gln Thr Leu Met
415 420 425
AAC TCC ATG GAC CCC GAG TCC ACA CCA CCC ACC TGC TGT GTG CCC ACG 2046
Asn Ser Met Asp Pro Glu Ser Thr Pro Pro Thr Cys Cys Val Pro Thr
430 435 440
CGG CTG AGT CCC ATC AGC ATC CTC TTC ATT GAC TCT GCC AAC AAC GTG 2094
Arg Leu Ser Pro Ile Ser Ile Leu Phe Ile Asp Ser Ala Asn Asn Val
445 450 455
GTG TAT AAG CAG TAT GAG GAC ATG GTC GTG GAG TCG TGT GGC TGC AGG 2142
Val Tyr Lys Gln Tyr Glu Asp Met Val Val Glu Ser Cys Gly Cys Arg
460 465 470
TAG CAGCACTGGC CCTCTGTCTT CCTGGGTGGC ACATCCCAAG AGCCCCTTCC 2195
***
475
TGCACTCCTG GAATCACAGA GGGGTCAGGA AGCTGTGGCA GGAGCATCTA CACAGCTTGG 2255
GTGAAAGGGG ATTCCAATAA GCTTGCTCGC TCTCTGAGTG TGACTTGGGC TAAAGGCCCC 2315
CTTTTATCCA CAAGTTCCCC TGGCTGAGGA TTGCTGCCCG TCTGCTGATG TGACCAGTGG 2375
CAGGCACAGG TCCAGGGAGA CAGACTCTGA ATGGGACTGA GTCCCAGGAA ACAGTGCTTT 2435
CCGATGAGAC TCAGCCCACC ATTTCTCCTC ACCTGGGCCT TCTCAGCCTC TGGACTCTCC 2495
TAAGCACCTC TCAGGAGAGC CACAGGTGCC ACTGCCTCCT CAAATCACAT TTGTGCCTGG 2555
TGACTTCCTG TCCCTGGGAC AGTTGAGAAG CTGACTGGGC AAGAGTGGGA GAGAAGAGGA 2615
GAGGGCTTGG ATAGAGTTGA GGAGTGTGAG GCTGTTAGAC TGTTAGATTT AAATGTATAT 2675
TGATGAGATA AAAAGCAAAA CTGTGCCT 2703

## Claims

1. A protein named HMW human MP52 which is selected from the group consisting of:
(1) a dimeric protein comprising peptides having amino acid sequence from 1 to 474 of SEQ ID NO.:1.
(2) a dimeric protein comprising peptides having amino acid sequence from 121 to 474 of SEQ ID NO.:1.
(3) a dimeric protein comprising peptides having amino acid sequence from 122 to 474 of SEQ ID NO.:1.
(4) a dimeric protein comprising a peptide having amino acid sequence from 121 to 474 of SEQ ID NO.:1 and a peptide having amino acid sequence from 122 to 474 of SEQ ID NO.:1.
(5) a dimeric protein comprising a peptide having amino acid sequence from 1 to 474 of SEQ ID NO.:1 and a peptide having amino acid sequence from 121 and/or 122 to 474 of SEQ ID NO.:1.
(6) a dimeric protein comprising a peptide having amino acid sequence from 1 to 474 of SEQ ID NO.:1 and a peptide having amino acid sequence from 355 to 474 of SEQ ID NO.:1.
(7) a dimeric protein comprising a peptide having amino acid sequence from 121 and/or 122 to 474 of SEQ ID NO.:1 and a peptide having amino acid sequence from 355 to 474 of SEQ ID NO.:1. and the mixture thereof.

2. A process for the production of HMW human MP52s defined in claim 1 which comprises introducing DNA containing DNA-sequence at least from 721 to 2145 as shown in SEQ ID NO.:1 into a suitable host cell and cultivating the host cell under conditions that allow expression of the DNA-sequence and protein formation.

3. A pharmaceutical composition containing as an active ingredient at least one of HMW human MP52s defined in claim 1.

4. The pharmaceutical composition as claimed in claim 3 for the use of orthopedic reconstruction, bone transplantation, cosmetic surgery or dental implantation.

5. Use of the pharmaceutical composition as claimed in claim 3 for promoting bone morphogenesis, treatment or prevention of damage to bone, cartilage, connective tissues, skin, mucous membranes, epithelium or teeth, for application in dental implants and for application in wound-healing and tissue regeneration processes.

6. Use of the pharmaceutical composition as claimed in claim 3 for treating osteoporosis or bone fracture.
